# EUROPÄISCHE PATENTSCHRIFT

(11) **EP 1 753 355 B1**
(45) Veröffentlichungstag und Bekanntmachung des Hinweises auf die Patenterteilung: **09.11.2011**
(21) Anmeldenummer: 04723488.5
(22) Anmeldetag: 26.03.2004
(51) Int. Cl.: A61B 17/86

(54) **KNOCHENSCHRAUBE MIT GELENK**
ARTICULATED BONE SCREW
VIS À OS AVEC ARTICULATION

(43) Veröffentlichungstag der Anmeldung: 21.02.2007
(73) Patentinhaber: Synthes GmbH, 4436 Oberdorf (CH)
(72) Erfinder: NIEDERBERGER, Alfred, CH-2540 Grenchen (CH); VAN DER WERKEN, Christian, NL-3723 AC (NL)
(74) Vertreter: Lusuardi, Werther
(86) Internationale Anmeldenummer: PCT/CH2004/000187
(87) Internationale Veröffentlichungsnummer: WO 2005/092226

(56) Entgegenhaltungen:
- DE-U- 9 300 056
- US-A1- 2002 198 527
- US-B1- 6 475 242

## Beschreibung

Die Erfindung bezieht sich auf eine Knochenschraube gemäss dem Oberbegriff des Patentanspruchs 1.

Aus der US 4,959,064 ENGELHARDT ist eine Knochenschraube mit einem Federteil offenbart. Der Federteil im Schaft der Knochenschraube verleiht ihr eine gewisse axiale Elastizität (axiale Kompression oder Distraktion) sowie eine gewisse Torsion und auch eine gewisse Biegung nach radial in allen Richtungen. Diese bekannte Knochenschrauben will somit nur einen Abfall der Kompressionswirkung der Schraube verhindern.

Aus der EP-A 1,273,269 MÜCKTER ist eine Knochenschraube mit elastischem Schaft offenbart. Die Elastizität dieser Konstruktion wie in diversen Ausführungsformen beschrieben. Auch hier verleiht die elastische Verbindung der Schraube eine gewisse axiale Elastizität. Eine Übertragung von Drehmomenten ist jedoch, ohne Zuhilfenahme weiterer stabilisierender Instrumente oder Implantate, nicht möglich.

Hier will die Erfindung Abhilfe schaffen. Der Erfindung liegt die Aufgabe zugrunde, eine Knochenschraube zu schaffen, welche relativ zu ihrer Längsachse in allen Richtungen abwinkelbar ist, unter gleichzeitiger Beibehaltung ihrer axialen Starrheit und voller Übertragung eines Drehmomentes ohne Zuhilfenahme zusätzlicher Mittel.

Die Erfindung löst die gestellte Aufgabe mit einer Knochenschraube, welche die Merkmale des Anspruchs 1 aufweist.

Die durch die Erfindung erreichten Vorteile sind im wesentlichen darin zu sehen, dass dank der erfindungsgemässen Knochenschraube Knochen derart miteinander verbunden werden können, dass eine gewisse Abwinkelung und damit Beweglichkeit zueinander ermöglicht wird.

Bei einer besonderen Ausführungsform besteht das Kardangelenk aus einem klassischen Kreuzgelenk. Bei einer anderen Ausführungsform besteht das kardanähnliche Gelenk aus einem Kugelgelenk mit einem Kugelkopf, welcher einen polygonalen, vorzugsweise achteckigen Querschnitt aufweist und einer Kugelschale, welche geeignet ist den Querschnitt des Kugelkopfes aufzunehmen. Der Vorteil dieser Ausführung mit einem Kugelachtkant ist die vereinfachte Auslegung der Konstruktion bei etwas eingeschränkter Freiheit gegenüber dem klassischen Kardangelenk.

Bei einer weiteren Ausführungsform weist die Knochenschraube mehrere Kardan- oder kardanähnliche Gelenke auf. Der Vorteil dieser Anordnung besteht im vergrösserten Freiheitsgrad der Knochenschraube.

Bei einer weiteren Ausführungsform weist die Knochenschraube eine koaxial zu ihrer Längsachse verlaufende, durchgehende Kannulierung auf. Dies gestattet eine Blockierung des Kardangelenkes durch Einführung eines Kirschnerdrahtes in die Kannulierung.

Vorteilhafterweise ist die Länge des Schaftes - gebildet aus der Summe der beiden Längen des proximalen Abschnitts und des distalen Abschnitts - konstant.

Bei einer bevorzugten Ausführungsform ist auch der proximale Abschnitt mindestens teilweise mit einem Aussengewinde versehen. Falls man einen bestimmten Abstand zwischen den Knochenteilen wahren will, kann man mit einem passenden Bohrer die Knochenteile aufbohren, so dass dann sowohl das Gewinde im distalen Abschnitt als auch dasjenige im proximalen Abschnitt greifen. Der Abstand zwischen den beiden Knochenteilen bleibt nun fest auf einen bestimmten Wert eingestellt. Auf diese Weise wird die Knochenschraube als Stellschraube eingesetzt.

Will man anderseits eine Abstandsänderung zwischen den beiden Knochenteilen erlauben, dann bohrt man die zur Aufnahme des proximalen Teils der Schraube bestimmte Bohrung mit einem grösseren Durchmesser als das Aussengewinde des proximalen Teils. Das proximale Gewinde greift jetzt nicht im proximalen Knochenteil (z.B. in der Clavicula). Die Knochenteile können jetzt ihren Abstand zueinander verändern. Dies natürlich nur innerhalb gewisser Grenzen, da der maximale Abstand durch den Schraubenkopf begrenzt wird. Die Knochenschraube wir somit in diesem Fall als Zugschraube verwendet.

Die Knochenschraube mit proximalem Gewinde lässt sich somit universaler verwenden. Will man beispielsweise erreichen, dass die Knochenteile zusammen konsolidieren, verwendet man sie als Zugschraube und schraubt sie beispielsweise bis zum Processus coracoideus.

Bei einer besonderen Ausführungsform ist die Knochenschraube selbstschneidend und/oder selbstbohrend ausgebildet.

Bei einer weiteren Ausführungsform ist die Auslenkung des Gelenke begrenzt, vorzugsweise auf maximal 90° und zweckmässigerweise auf maximal 30°.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht der Knochenschraube mit einem Kardangelenk und mit eingeführtem Kirschnerdraht;
Fig. 2 einen Längsschnitt durch die Knochenschraube im geradlinig ausgerichteten Zustand ohne Kirschnerdraht;
Fig. 3 eine perspektivische Ansicht der Knochenschraube nach Fig. 2 im abgewinkelten Zustand ohne Kirschnerdraht;
Fig. 4 einen partiellen Längsschnitt durch ein modifiziertes kardanähnliches Kugelgelenk für eine Knochenschraube;
Fig. 5 eine Ansicht der im Schulterbereich implantierten Knochenschraube nach den Fig. 1 - 3; und
Fig. 6 eine Ansicht der im Sprunggelenkbereich implantierten Knochenschraube nach den Fig. 1-3.

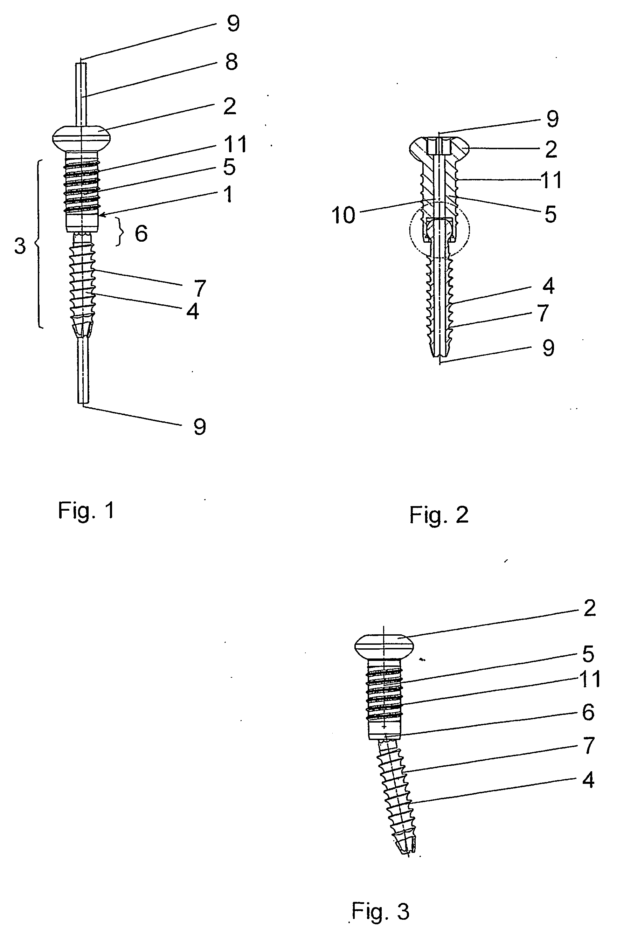

Die in den Figuren 1-3 dargestellte erfindungsgemässe Knochenschraube 1 besitzt einen Kopfteil 2, einen Schaft 3, eine Längsachse 9 und eine durchgehende zentrale Kannulierung 10. Der Schaft 3 besteht aus einem an den Kopfteil 2 angrenzenden proximalen Abschnitt 5 mit Aussengewinde 11 und einem mittels eines KardanGelenkes 6 daran befestigten, zur Einführung in den Knochen geeigneten, distalen Abschnitt 7 mit einem Aussengewinde 4. Wird in die zentrale Kannulierung 10 (Fig. 2) ein Kirschnerdraht 8 (Fig. 1) eingeführt erfolgt eine Blockierung des Kardangelenkes 6, so dass die Knochenschraube 1 nicht mehr - wie in Fig. 1 angedeutet - abgewinkelt werden kann.

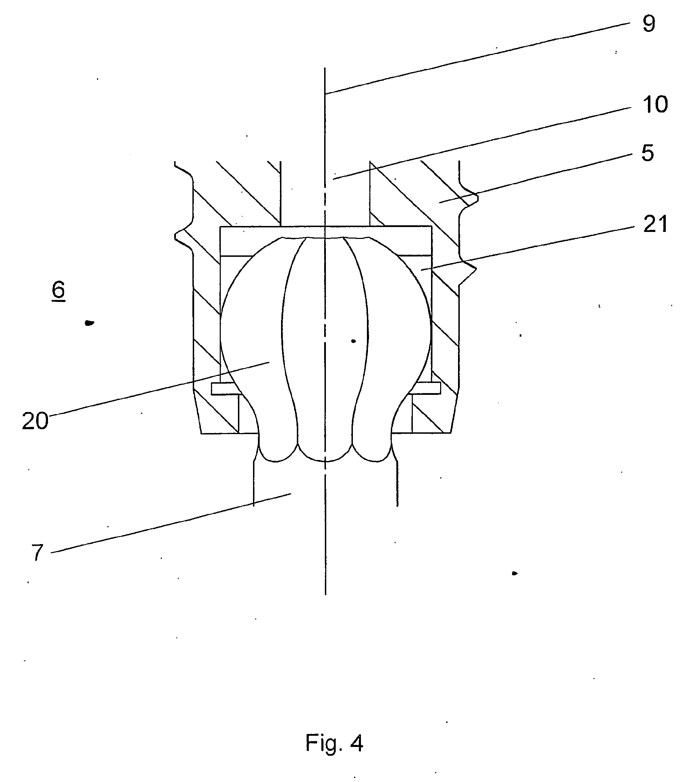

Wie in Fig. 4 im Detail dargestellt besteht das Kardangelenk 6 aus einem Kugelgelenk mit einem Kugelkopf 20 und einer im proximalen Abschnitt 5 untergebrachten Kugelschale 21. Der Kugelkopf 20 weist einen achteckigen Querschnitt auf und die Kugelschale 21 besitzt eine entsprechend angepasste, achteckige Geometrie, welche geeignet ist den Querschnitt des Kugelkopfes 20 aufzunehmen. Diese Geometrie gestattet eine Rotation der Knochenschraube 1 auch im abgewinkelten Zustand. Statt eines Kardangelenkes kann auch ein klassisches Kreuzgelenk verwendet werden.

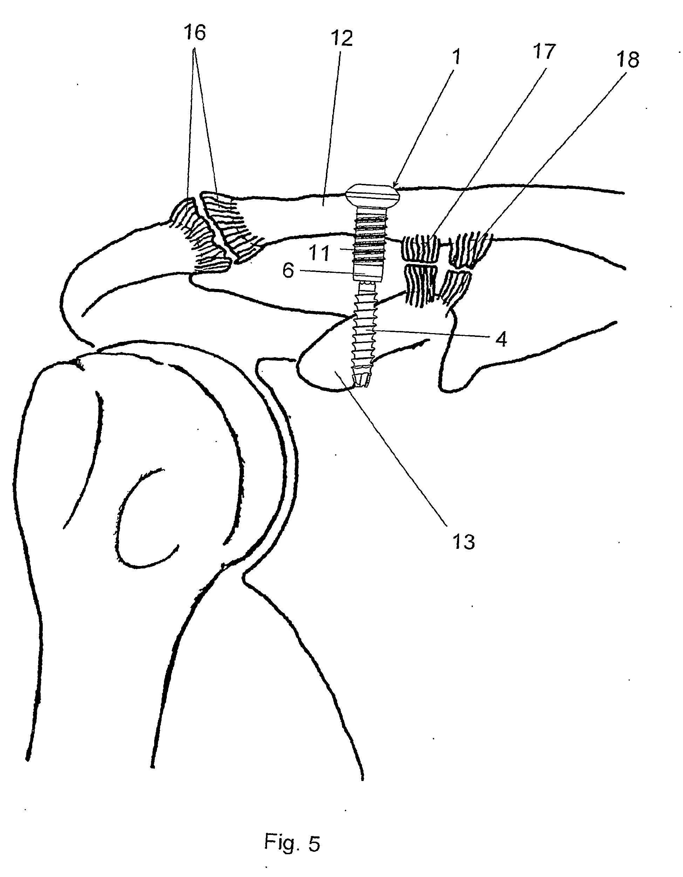

In Fig. 5 ist eine Anwendung der erfindungsgemässen Knochenschraube 1 im Schulterbereich dargestellt, um eine Verbindung zwischen der Clavicula 12 und dem Processus coracoideus 13 herzustellen. Normalerweise wird die Clavicula 12 von den Ligamenten Lig. acromiclaviculare 16, Lig. trapezoidum 17 und dem Lig. conoideum 18 in Position gehalten. Tritt eine Ruptur dieser Ligamente auf, wird die Clavicula 12 nicht mehr in Position gehalten und der sogenannte "Ktaviertasteneffekt" tritt auf. Dabei wird die Clavicula 12 durch Muskeln nach oben gezogen und steht dann im Bereich des Schultergelenkes ab. Die operative Behandlung nach dem Stand der Technik besteht darin, dass man die Ligamente zusammennäht und während einer gewissen Zeit (mehrere Monate lang) eine rigide Knochenschraube einbringt, damit keine Kräfte auf die Ligamente wirken. Leider brechen rigide Knochenschrauben unter den gegebenen Bedingungen. Die Verwendung der erfindungsgemässen Knochenschraube 1 (statt einer rigiden Schraube) erlaubt die Verbindung der gegeneinander beweglich gewordenen Clavicula 12 und Processus coracoideus 13, so dass die zusammengenähten Ligamente 16,17,18 entlastet bleiben. Eine Abwinkelung der beiden Knochen wird dabei durch das Kardangelenk 6 der Knochenschraube 1 zugelassen; gleichzeitig wird aber der Abstand zwischen den beiden Knochen beibehalten.

Die Operationstechnik für diese Anwendung im Schulterbereich wird nachstehend kurz beschrieben:
a) ein Kirschnerdraht 8 wird durch die Clavicula 12 hindurch in den Processus coracoideus 13 eingedreht;
b) über den Kirschnerdraht 8 wird mittels eines durchbohrten Bohrers ein Durchgangsloch durch die Clavicula 12 gebohrt. Der Processus coracoideus 13 wird hingegen nicht vorgebohrt;
c) die Knochenschraube 1 wird über den Kirschnerdraht 8 in das in der Clavicula 12 vorgebohrte Loch eingebracht und mit ihrem Aussengewinde 4 in den Processus coracoideus 13 eingedreht. Der Kirschnerdraht 8 stabilisiert dabei die Knochenschraube 1 in diesem Moment und führt sie zugleich; und
d) der Kirschnerdraht 8 wird entfernt und die Knochenschraube 1 wird dadurch abwinkelbar.

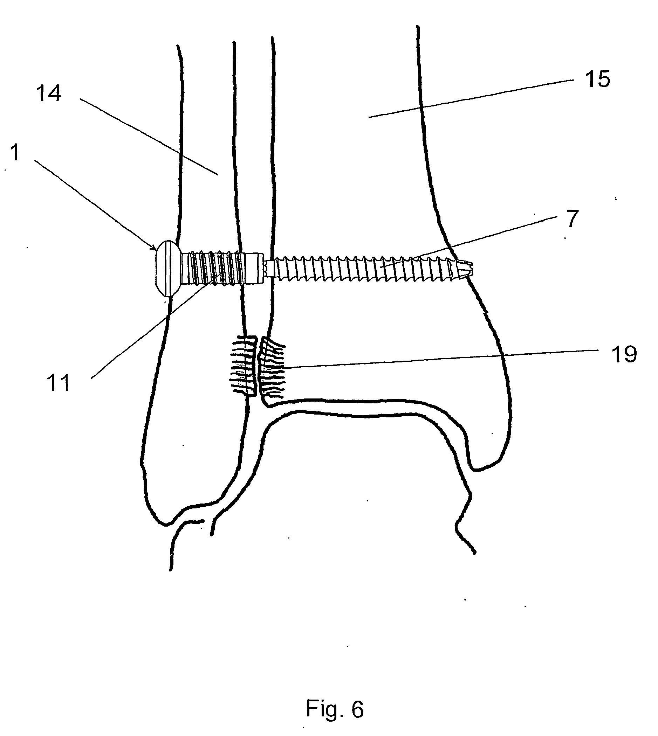

In Fig. 6 ist eine weitere Anwendung der erfindungsgemässen Knochenschraube 1 im Bereich des Sprunggelenkes dargestellt, um eine Verbindung der Fibula 14 mit der Tibia 15 herzustellen. Das zwischen der Fibula 14 und der Tibia 15 vorhandene Band 19 (Syndesmose) ist gerissen und, so dass Fibula 14 und Tibia 15 auseinanderdriften. Gemäss dem Stand der Technik wird das Band 19 genäht. Durch Verwendung der efindungsgemässen Knochenschraube 1 kann das genähte Band 19 entlastet werden unter Beibehaltung des Abstandes der beiden Knochen. Die Operationstechnik ist dabei analog zu derjenigen, welche anhand der Fig. 5 beschrieben worden ist.

## Patentansprüche

1. Knochenschraube (1) mit einem Kopfteil (2), einem Schaft (3) und einer Längsachse (9), wobei
der Schaft (3) aus einem an den Kopfteil (2) angrenzenden proximalen Abschnitt (5) und einem daran befestigten, zur Einführung in den Knochen geeigneten, distalen Abschnitt (7) besteht, der mindesten teilweise mit einem Aussengewinde (4) versehen ist **dadurch gekennzeichnet, dass** der proximale Abschnitt (5) und der distale Abschnitt (7) mittels eines Kardan- oder kardanähnlichen Gelenkes (6) miteinander verbunden sind.

2. Knochenschraube (1) nach Anspruch 1, **dadurch gekennzeichnet, dass** das KardanGelenk (6) aus einem klassischen Kreuzgelenk besteht.

3. Knochenschraube (1) nach Anspruch 1, **dadurch gekennzeichnet, dass** das kardanähnliche Gelenk (6) aus einem Kugelgelenk mit einem Kugelkopf (20), welcher einen polygonalen, vorzugsweise achteckigen Querschnitt aufweist und einer Kugelschale (21), welche geeignet ist den Querschnitt des Kugelkopfes (20) aufzunehmen.

4. Knochenschraube (1) nach einem der Ansprüche 1 bis 3, **dadurch gekennzeichnet, dass** sie mehrere Kardan- oder kardanähnlichen Gelenke (6) aufweist.

5. Knochenschraube nach einem der Ansprüche 1 bis 4, **dadurch gekennzeichnet, dass** sie eine koaxial zur Längsachse (9) verlaufende durchgehende Kannulierung (10) aufweist.

6. Knochenschraube nach einem der Ansprüche 1 bis 5, **dadurch gekennzeichnet, dass** die Länge des Schaftes (3) gebildet aus der Summe der beiden Längen des proximalen Abschnitts (5) und des distalen Abschnitts (7), konstant ist.

7. Knochenschraube nach einem der Ansprüche 1 bis 6, **dadurch gekennzeichnet, dass** auch der proximale Abschnitt (5) mindestens teilweise mit einem Aussengewinde (11) versehen ist.

8. Knochenschraube nach einem der Ansprüche 1 bis 7, **dadurch gekennzeichnet, dass** sie selbstschneidend und/oder selbstbohrend ausgebildet ist.

9. Knochenschraube nach einem der Ansprüche 1 bis 8, **dadurch gekennzeichnet, dass** die Auslenkung des Gelenke (6) begrenzt ist, vorzugsweise auf maximal 90°.

10. Knochenschraube nach einem der Ansprüche 3 bis 8, **dadurch gekennzeichnet, dass** die Auslenkung des als Kugelgelenk ausgebildeten Gelenkes (6) begrenzt ist, vorzugsweise auf maximal 30°.

## Claims

1. Bone screw (1) with a head part (2), a shaft (3) and a longitudinal axis (9), wherein
the shaft (3) comprises a proximal section (5), adjoining the head part (2) and a distal section (7) for introduction into the bone, which is attached to the proximal section (5) and which is provided at least partially with an external thread (4),
**characterized in that**
the proximal section (5) and the distal section (7) are connected to each other by means of a cardan or cardan-like joint (6).

2. The bone screw (1) of claim 1, **characterized in that** the cardan joint (6) consists of a classical universal joint.

3. The bone screw (1) of claim 1, **characterized in that** the cardan-like joint (6) comprises a ball joint with a ball head (20), which has a polygonal, preferably octagonal cross section and a ball socket (21) which is suitable for accommodating the cross section of the ball head (20).

4. The bone screw (1) of one of the claims 1 to 3, **characterized in that** it has several cardan or cardan-like joints (6).

5. The bone screw of one of the claims 1 to 4, **characterized in that** it has a continuous cannulation (10), which extends coaxially with the longitudinal axis (9).

6. The bone screw of one of the claims 1 to 5, **characterized in that** the length of the shaft (3), formed from the sum of the two lengths of the proximal section (5) and the distal section (7), is constant

7. The bone screw of one of the claims 1 to 6, **characterized in that** the proximal section (5) is also provided at least partially with an external thread (11).

8. The bone screw of one of the claims 1 to 7, **characterized in that** it is constructed to be self-cutting and/or self-drilling.

9. The bone screw of one of the claims 1 to 8, **characterized in that** the deflection of the joint (6) is limited preferably to a maximum of 90°.

10. The bone screw of one of the claims 3 to 8, **characterized in that** the deflection of the joint (6), constructed as a ball joint, is limited preferably to a maximum of 30°.

## Revendications

1. Vis à os (1) comprenant une partie (2) formant la tête, une tige (3) et un axe longitudinal (9), la tige (3) se composant d'une partie proximale (5) contiguë à la partie (2) formant la tête et d'une partie distale (7) fixée sur cette partie proximale (5) et propre à l'introduction dans l'os, laquelle partie distale (7) est dotée au moins partiellement d'un filetage extérieur (4),
**caractérisée en ce que** la partie proximale (5) et la partie distale (7) sont liées l'une à l'autre au moyen d'une articulation à cardan (6) ou semblable à un cardan.

2. Vis à os (1) selon la revendication 1, **caractérisée en ce que** l'articulation à cardan (6) se compose d'un joint à croisillon classique.

3. Vis à os (1) selon la revendication 1, **caractérisée en ce que** l'articulation (6) semblable à un cardan se compose d'un joint à rotule comprenant une tête sphérique (20) qui présente une section polygonale, de préférence octogonale, et d'une cuvette sphérique (21) qui est appropriée pour loger la section de la tête sphérique (20).

4. Vis à os (1) selon l'une des revendications 1 à 3, **caractérisée en ce qu'**elle comporte plusieurs articulations à cardan (6) ou semblables à un cardan.

5. Vis à os selon l'une des revendications 1 à 4, **caractérisée en ce qu'**elle comporte un canal continu (10) s'étendant de façon coaxiale par rapport à l'axe longitudinal (9).

6. Vis à os selon l'une des revendications 1 à 5, **caractérisée en ce que** la longueur de la tige (3), formée par la somme des deux longueurs de la partie proximale (5) et de la partie distale (7), est constante.

7. Vis à os selon l'une des revendications 1 à 6, **caractérisée en ce que** la partie proximale (5) est dotée aussi, au moins partiellement, d'un filetage extérieur (11).

8. Vis à os selon l'une des revendications 1 à 7, **caractérisée en ce qu'**elle est configurée en étant autotaraudeuse et/ou autoforeuse.

9. Vis à os selon l'une des revendications 1 à 8, **caractérisée en ce que** la déviation de l'articulation (6) est limitée, de préférence au maximum à 90°.

10. Vis à os selon l'une des revendications 3 à 8, **caractérisée en ce que** la déviation de l'articulation (6) configurée comme un joint à rotule, est limitée, de préférence au maximum à 30°.
